# EUROPEAN PATENT SPECIFICATION

(11) **EP 0 621 784 B1**
(45) Date of publication and mention of the grant of the patent: **04.06.1997**
(21) Application number: 93903184.5
(22) Date of filing: 20.01.1993
(51) Int. Cl.: A61K 33/26

(54) **AN IRON-CONTAINING COMPOSITION FOR THE PREVENTION OF ANAEMIA AND A METHOD FOR PRODUCING THE COMPOSITION**
EISENHALTIGE ZUSAMMENSETZUNG ZUR VERHÜTUNG VON ANÄMIE UND VERFAHREN ZUR HERSTELLUNG DER ZUSAMMENSETZUNG
COMPOSITION CONTENANT DU FER SERVANT A LA PREVENTION DE L'ANEMIE ET PROCEDE DE PREPARATION

(30) Priority: 20.01.1992 DK 64/92; 17.12.1992 WO PCT/DK92/00384
(43) Date of publication of application: 02.11.1994
(73) Proprietor: HOLM CHRISTENSEN, Borge, DK-3140 Aalsgaarde (DK)
(72) Inventor: HOLM CHRISTENSEN, Borge, DK-3140 Aalsgaarde (DK)
(74) Representative: Plougmann, Vingtoft & Partners A/S
(86) International application number: DK9300017
(87) International publication number: WO9313783

(56) References cited:
- DK-B- 136 101
- GB-A- 1 409 468
- GB-A- 1 465 781
- SE-B- 466 287
- Dialog Information Service, File 351, WPIL, Dialog Accession Nr. 00824871, WPI Accession No. 90-135712/18 (SNOW BRAND MILK PRODUCTS), "Iron oasein prepn., useful in treatment of anaemia - by reacting"; & JP-A-02 083 400

## Description

### FIELD OF INVENTION

The present invention relates to the prevention and treat ment of anaemic conditions in animals and humans and there is provided an oral composition containing a compound containing iron in a highly bioavailable form which composition has a palatability allowing it to be ingested voluntarily by newborn, suckling individuals in amounts sufficient to cover their physiological requirements for iron. The composition according to the invention may also include substances such as dietary fiber-containing ingredients which prevent or cure diarrhoea including scouring in young animals. Furthermore there is provided a method of producing an iron-containing composition according to the invention.

### TECHNICAL BACKGROUND AND PRIOR ART

Anaemic conditions may occur as a result of losses of blood from an individual or as a result of insufficient supply of bioavailable iron in the diet. In this respect, a particular problem exists in the pig industry. Newborn piglets have a total body reservoir of iron which is about 50 mg. During the first 2-3 weeks of life (the suckling period) their weight gain is so rapid that the daily requirement for iron is about 7-10 mg in order to maintain a physiologically normal level of haemoglobin in their blood, i.e. about 90-120 g per l. However, the daily supply of iron from the sow's milk is only about 1 mg and inevitably, a serious and often fatal, anaemic condition will occur within a few days after birth, if a supplementary iron supply is not provided.

Presently, such a supplementary supply of iron is normally provided by giving newborn piglets an injection of an iron-containing substance such as an iron dextran. Obviously, this method of supplying iron is very labour-consuming and in addition it involves a risk of spreading infectious microorganisms via the injection needle and of causing a stress condition in the animals.

It is therefore not surprising that several attempts have been made to provide iron-containing compositions which by oral administration to suckling piglets might supplement their insufficient supply of iron.

A general problem associated with oral administration to suckling animals of iron-containing compositions is the fact that their energy requirements is substantially covered via the mother's milk and hence they show no willingness to ingest more than a few grammes of solid sources of nutrients and in addition, suckling animals are extremely selective with regard to which solid materials they are willing to ingest.

Sources of iron in oral compositions may be organic or inorganic iron salts or compounds containing chelated iron or complex-bound iron. When selecting a suitable iron source for an oral composition, several factors must be taken into account. Whereas it may be advantageous to include an iron source with a high solubility and hence a high degree of bioavailability, at the conditions prevailing in the gastrointestinal tract in order to provide a required dosage at a low amount of the iron-containing composition, such a high solubility may at the same time render the composition so unpalatable that a voluntary ingestion in sufficient amounts by suckling animals, is not obtainable.

Alternatively, it may be attempted to include an iron source having a low solubility and a low degree of bioavailability under gastrointestinal conditions, including those of the oral cavity, whereby the iron-containing composition might become more palatable. In general, however, such an approach would require the administration of amounts of the iron-containing composition exceeding those which may be voluntarily ingested by suckling animals in order to provide a physiologically sufficient iron dosage. In addition, even compositions containing such sparingly soluble iron compounds may not have an acceptable palatability for the suckling animals.

In DE 21 11 638 is disclosed a composition containing iron polysaccharide complexes which is recommended for oral administration to suckling piglets during the first day of life. However, the composition has to be administered by introduction of the composition into the oral cavity in the form of a suspension (by use of a pistol), tablets, capsules, a paste or an aerosol.

There has also been introduced solid iron-containing compositions, e.g. containing ferrous fumarate, which may be ingested voluntarily by suckling animals. However, these known compositions contain iron which is in a form where less than 10% is absorbable and furthermore, in order to provide these known compositions in a sufficiently palatable form, it is required to incorporate relatively low concentrations of iron, such as 10 wt%. Accordingly, a suckling piglet must ingest about 80 grammes during the first two weeks of life in order to avoid anaemia. A number of suckling piglets may not be willing to ingest such a high amount of the composition.

In this context, it is also likely that the requirement to administer the above rather high amounts of an oral iron-supplementing composition is due to an inappropriately large particle size of the selected iron compound. It is known that the iron bioavailability, i.e. the proportion of the iron which can be absorbed from the intestinal mucosal membranes, depends on several factors including the intestinal pH and the particle size. In suckling piglets, the intestinal pH is typically in the range of 4.5 to 6.5. Within this pH range, the solubility of several organic iron compounds including ferrous fumarate is relatively low. Therefore, the incorporation of sparingly soluble iron salts in the form of large particles may result in a low bioavailability of iron in suckling animals.

This problem of reduced bioavailability may be solved by providing the iron in the form of compounds having a small particle size. However, the use of ferrous compounds which are preferred since their bioavailability is generally higher than that of corresponding ferric compounds as small particles in the form of small particles gives rise to a new problem, viz. an acceleration of oxidation of the ferrous compound to ferric compound.

As an alternative to the above direct oral administration to suckling animals of iron-containing compositions it has been suggested to supply a solution of an organic iron compound via automated drinking systems and it has been found that adequate amounts of iron may be taken up by the animals via this route of administration. However, it is required to clean the drinking systems on a daily basis and besides, there is a considerable waste of the iron-containing preparation which may add unacceptably to the costs of such an administration.

It is obvious from the above that a need exists to provide a solid iron-containing composition for the prevention and treatment of anaemic conditions, in particular in suckling animals, which can be administered orally without any manipulation of individual animals and which cost-effectively secures a physiologically normal haemoglobin concentration during periods where the normal dietary iron supply is inadequate.

The present invention provides such an oral iron-containing composition which comprises the iron in a highly bioavailable form and which provides the iron in a palatable form so as to allow the composition to be voluntarily ingested by suckling animals including piglets, in sufficient amounts to maintain the physiologically normal level of haemoglobin.

### SUMMARY OF THE INVENTION

Accordingly, the present invention relates in a first aspect to a composition containing gastrointestinally absorbable iron, comprising 0.1 to 25 wt% of elemental iron and 1 to 99 wt% of an amino acid-containing ingredient, the composition being in the form of a free-flowing powder of particles comprising a continuous coating layer containing the amino acid-containing ingredient and an inner core surrounded by the coating layer.

In a further aspect, the present invention pertains to a method of producing a free-flowing powdered iron-containing composition comprising particles of an inner core carrier material coated with a continuous layer of a coating composition comprising an amino acid-containing ingredient, the method comprising
(i) preparing a liquid coating composition comprising an amino acid-containing ingredient in an amount in the range of 1 to 99 wt%, calculated on the iron-containing composition,
(ii) providing a particulate carrier material having an average particle size being in the range of 1 µm to 100 µm,
(iii) adding to the coating composition and/or the inner core carrier material an iron compound in an amount providing a content of elemental iron which is in the range of 0.1 to 25 wt%, calculated on the iron-containing composition,
(iv) supplying the coating composition in liquid form to the atomizing means of a spray-drying plant comprising a spray-drying chamber, and atomizing the liquid coating composition into a flow of droplets,
(v) supplying a flow of transport gas comprising particles of the carrier material dispersed therein to the spray-drying chamber separately from the coating composition,
(vi) supplying a flow of drying gas to the chamber at a temperature which tends to solidify the liquid coating composition,
(vii) allowing droplets in the flow of liquid droplets of the coating composition to collide with the particles of the carrier material dispersed in the transport gas, the direction and rate of flow of the transport gas being adapted to substantially prevent contact between on one hand the drying gas and on the other hand the droplets, so that the liquid coating composition, before any substantial drying thereof, will form a substantially continuous liquid layer on the carrier material particles,
(viii) then allowing the thus applied continuous coating layer on the particles to at least partially dry by contact with the drying gas, and
(ix) withdrawing the coated particles from the spray-drying chamber.

In a still further aspect, the composition according to the invention provides means for a method of dietetically supplying iron to an individual suffering from anaemia, comprising administering to said individual the composition as defined herein in an amount and for a period of time which results in a concentration of haemoglobin in the blood of the individual of at least 90 g per l.

### DETAILED DISCLOSURE OF THE INVENTION

As it is mentioned above, the present invention provides a free-flowing powdered composition containing gastrointestinally absorbable iron. The composition is generally suitable as a dietary iron supplementation means in individuals suffering from an anaemic condition, but it is particularly useful in newborn, suckling animals such as piglets as an easily administrable iron supplement source. The composition is preferably provided as particles having an average size which is at the most 1 mm such as at the most 0.5 mm.

The source of elemental iron may be any iron compound which when present in the composition as defined herein and administered as defined below, results in the maintenance of a physiologically normal haemoglobin of an animal to which it is administered. In the present context, a suitable iron compound may be selected from an organic iron salt, an inorganic iron salt and a compound in which the iron is chelated or present in a complex-bound form. Although ferrous compounds may be preferred due to a higher bioavailability relative to ferric compounds, the latter group of compounds may also be suitable. Inorganic compounds which may be suitable include as examples iron salts of hydrochloric and sulphuric acid.

In accordance with this invention, any organic iron compound which has the above effect may be selected. In preferred embodiments, the composition comprises an organic iron compound which preferably may be one selected from a formate, an acetate, a propionate, a fumarate, a lactate, a citrate, a succinate, a fatty acid salt of iron, an amino acid salt of iron including glutamate or there may be used a mixture of two or more of these salts. Other useful iron-containing substances include substances wherein the iron is complex-bound to a carbohydrate moiety such as a dextran, and haeme which is the iron-containing prostestic group found in haemoglobin and myoglobin and which may be obtained from these proteins by removing the globin moiety. As it has been mentioned, ferrous compounds are generally more preferred than ferric compounds, since ferrous compounds are more readily absorbed from the intestines.

Commercially available qualities of organic iron compounds may be in the form of relatively coarse particles. As it is mentioned above, iron salts such as e.g. ferrous fumarate which at the pH conditions prevailing in the gastrointestinal tract of suckling animals are sparingly soluble may have an unacceptably low bioavailability when provided as large particles, i.e. particles of a size exceeding 50 µm. Accordingly, in preferred embodiments the present composition may contain an organic iron salt which is in the form of particles of an average size which is at the most 50 µm, such as at the most 30 µm and preferably at the most 10 µm.

In the present context, one particularly useful and economically feasible iron salt is ferrous fumarate. Commercially available grades of this salt, however, may be in the form of particles, the average size of which is considerably larger than 50 µm. Therefore, when ferrous fumarate is selected as the organic iron salt, an initial step of preparing this salt in a finely grained or form is advantageously included in the preparation of the composition. This is conveniently carried out based on the process disclosed in US 3,478,073 according to which an aqueous mixture of fumaric acid and ferrous hydroxide is reacted under conditions where the precipitation of ferrous fumarate results in the formation of fine particles of the above defined size.

An other suitable and economically feasible organic iron source is ferrous formate which in contrast to ferrous fumarate is readily soluble. It has not hitherto been possible to apply this useful iron source in sufficient amounts in oral iron supplementing compositions for suckling animals due to the adverse taste of the dissolved salt. However, when ferrous formate is contained in the present composition, the dissolution of the salt in the oral cavity is avoided or retarded and accordingly, suckling animals may be willing to ingest a composition according to the present invention, in a sufficient amount to cover their need for iron, even if the iron salt is ferrous formate.

In certain preferred embodiments of the present composition, the iron is supplied in the form of salts of amino acids such as e.g. glutamates, or as iron bound to peptides. A mixed amino acid iron salt may thus be prepared by mixing a water soluble iron salt with a hydrolysate of protein whereby amino acid salts and salts of peptides are formed.

As it is mentioned above, the present composition has a content of elemental iron which is in the range of 0.1 to 25 wt%, calculated on the composition. The required amount of the iron-containing compound depends i.a. on the iron content of the compound, the solubility of the compound under the conditions prevailing in the gastrointestinal tract and the particle size in which the compound is provided.

From a cost point of view it is advantageous to select an iron compound with a high content of iron. As one example, ferrous chloride has an iron content of about 65 wt%. Among organic iron salts, a particularly high content of iron is found in ferrous formate (38 wt%), but also ferrous acetate, fumarate, succinate and malate have a high iron content (32-33 wt%). Ferrous glutamate has an iron content of about 28 wt% and a mixture of amino acid salts prepared as mentioned above may typically have an iron content of about 20 wt%.

The iron-containing compound is preferably one which, when present in the gastrointestinal tract has a high degree of bioavailability. Accordingly, when the iron source is an organic iron compound, a bioavailability which is at least 5% is preferred, although one having a bioavailability of at least 10% is more preferred. In particularly preferred embodiments, the organic iron salt has a bioavailability of at least 20%, such as at least 25%. When the iron source is an inorganic iron compound the bioavailability may preferably be at least 25%, more preferably at least 50% and most preferably at least 90%.

In preferred embodiments of the composition according to the present invention, the content of iron is in the range of 2 to 20 wt%, and even more preferably in the range of 3 to 10 wt%.

An essential characteristic of the present composition is that it has a palatability which allows for voluntary uptake by suckling animals in amounts providing a dosage which is sufficient to provide adaequate iron supplementation when administered as defined above. This characteristic is e.g. achieved by providing a dry composition comprising the iron compound embedded in or coated by an amino acid containing ingredient, the content of which is in the range of 1 to 99 wt%, calculated on the composition. As it has been mentioned above, ferrous iron e.g. when contained in organic compounds in the form of microscopic particles as defined above are prone to oxidation to the less bioavailable ferric iron. It has been found that such an oxidation may be significantly avoided by incorporating the organic iron compound into the amino acid containing ingredient-containing composition. It will be understood that ferrous iron contained in inorganic compounds will also be prone to oxidation to ferric iron.

Furthermore, this incorporation of iron serves the advantageous purpose of protecting readily soluble iron compounds from rapid dissolution in the oral cavity or further down in the gastrointestinal tract. It is known that the administration to a newly born animal of an iron salt which is readily soluble in the gastrointestinal tract may have a toxic or even lethal effect. Assumingly, this effect is due to absorption of more iron than the iron-transporting blood component transferrin is capable of binding. It has been found that the present iron composition can be administered to suckling animals even during the first days of life without any toxic effect.

A further significant advantage may be obtained by the provision of the iron compound embedded in or coated by an amino acid-containing ingredient in the form of a mixture of amino acids and peptides e.g. as a protein hydrolysate, since such low molecular nitrogen compounds may enhance the intestinal absorption of iron significantly. In addition to these advantages of using a protein hydrolysate in the present composition, the hydrolysate may also provide a dietetically valuable source of amino acids for the individuals to which the composition is administered.

The amino acid containing ingredient may be an animal or vegetable protein or it may be derived from animal or vegetable protein starting materials, although animal proteins may generally be more preferred due to their higher content of essential amino acids as compared to vegetable proteins. However, an amino acid containing ingredient which is one having a dietetically less optimal amino acid composition may be used, preferably in connection with the addition to the composition of a supplement of essential amino acids including lysine, methionine, threonine and tryptophane. Such a supplementation may also be required when an animal protein is used as the amino acid-containing ingredient or as starting material therefor, e.g. when the composition is intended for use in a suckling animal. In the present context, examples of suitable proteins include blood, haemoglobin, myoglobin, milk proteins including caseins and whey proteins, soy protein, leguminous seed proteins, meat and bone meal and fish protein in the form of fish meal. Such proteins may be used as the native proteins or they may be in the form of partially hydrolysed proteins.

One particularly useful amino acid-containing ingredient may be a protein hydrolysate including a hydrolysate of haemoglobin. Haemoglobin is available from animal slaughtering in large quantities at an acceptable cost level. A haemoglobin hydrolysate also provides an additional source of iron in a form which is generally considered to be highly bioavailable. It was surprisingly found during the experimentation leading to the present invention that the feeding to suckling piglets of a milk replacement product containing non-hydrolysed haemoglobin, in an amount which should provide an adequate iron supply, resulted in the development of anaemia in the piglets. It was hypothesized that this lack of utilization of haemoglobin iron might be a consequence of the deficiency in suckling animals of digestive enzymes capable of degrading non-milk proteins. This hypothesis was verified by carrying out a study in which native haemoglobin in the milk replacer was replaced by haemoglobin subjected to a partial enzymatic degradation. Feeding of the thus altered milk replacer to suckling piglets resulted in the maintenance of a physiologically normal level of haemoglobin.

Accordingly, in one advantageous embodiment of the present invention the amino acid-containing ingredient is partially hydrolysed haemoglobin. As an example, hydrolysed haemoglobin may be obtained by treating haemoglobin with an alkaline protease such as Carlsberg subtilisin at a temperature in the range of 50 to 70°C for 1 to 8 hours to provide a hydrolysate of haemoglobin in which at least 5% of the peptide bonds are hydrolysed, such as e.g. at least 10%. Other suitable proteolytically active enzymes include as examples trypsin, pepsin, chymosin and papain may in accordance with the invention, be used. Acid or alkaline hydrolysis may be used as well.

The content of the amino acid-containing ingredient in the composition is preferably in the range of 5 to 30 wt%, calculated on the composition, such as in the range of 10 to 25 wt%.

In accordance with the present invention it may be advantageous to incorporate a fatty acid-containing substance in the coating layer of the composition in an amount which is in the range of 1 to 50 wt%, calculated on the composition. Preferably, the amount of fatty acid-containing substance is in the range of 5 to 20 wt%, calculated on the composition. It is contemplated that the incorporation of a suitable fatty acid-containing substance in the composition may confer improved oxygen barrier characteristics to the composition by providing a coherent and continuous structure when the composition is dried. Furthermore, it is considered that the fatty acid-containing substance contributes to the prevention of an undesired oral cavity dissolution of the ferrous compound.

Preferred fatty acid-containing substances are substances which have a high degree of digestibility including as examples rendered lard, milk fat, hydrogenated vegetable oils and tallow. It is also contemplated that mixtures of fatty acid substances with melting point above ambient temperature with substances having melting point below this temperature may be useful. Such low melting point substances include vegetable and marine animal oils such as fish oil having a high content of n-3 unsaturated fatty acids. In one preferred embodiment, the composition is an emulsified mixture of an amino acid-containing ingredient such as hydrolysed protein and the fatty acid-containing substance into which the iron compound and optionally one or more of the further ingredients as mentioned below may be mixed. In order to enhance the emulsification, a suitable surface-active emulsifying agent may be added. Suitable emulsifying agents may be selected from commercial food grade emulsifying agents such as fatty acid esters or lecithin.

The iron-containing composition as defined herein may, in addition to those ingredients mentioned above contain at least one further ingredient. Such further ingredients include dietary fiber-containing ingredients, flavouring agents, vitamins, micronutrients, electrolytes, carbohydrates, bacterial cultures, enzymes, alkaline substances or acids. It may thus be particularly interesting to include one or more beneficial bacterial cultures having a growth promoting effect and/or a disease-preventing effect. In this context, useful bacteria may be Bacillus spp which produce exoenzymes capable of degrading nutrient components such as proteins, polysaccharides or fat. Other useful bacteria may be lactic acid-producing organisms such as Lactobacillus spp, Lactococcus spp, Bifidobacterium spp or Streptococcus spp. Interesting enzymes may be ones which degrade proteins (proteases), polysaccarides including starch and cellulose (amylases, cellulases) and/or fatty acid substances (lipases). Supplementation of the composition with such enzymes may be useful in compositions intended for suckling and weaning animals due to the above-mentioned deficiency in such animals of digestive enzymes capable of degrading the above macromolecules.

The present composition is typically provided in the form of a free-flowing powder having an average particle size which is at the most 1000 µm, e.g. in the range of 10 to 500 µm, preferably in the range of 50 to 250 µm such as e.g. in the range of 100 to 200 µm. In certain useful embodiments the composition may be one wherein a plurality of primary particles as defined herein are agglomerated.

In farm animal production diarrhoea or scouring in young animals is an other serious problem which is presently prevented or treated by the use of antibiotics. However, a widespread use of antibiotics in animals is associated with a strongly undesirable selection of antibiotic resistant bacteria which may be transferred to humans or healthy animals. Therefore, alternative means of prevention of scouring is needed. One known method is the administration of dietary fiber-containing compositions to young animals. In this context, the term "dietary fiber" is used to designate carbohydrate moieties which are not degradable by animal or human digestive enzymes. For the present invention, pectin-containing substances are presently considered to be particularly useful.

However, the use of such beneficial dietary fiber-containing compositions in suckling animals who are particularly prone to gastrointestinal infection is seriously restricted by the fact that these animals are not willing to ingest the compositions in the amounts which are required to prevent diarrhoea. It is contemplated that a major reason for this rejection is the readiness with which dietary fibers swell in the oral cavity due to their high water absorption capacity.

It has now been found that this problem may conveniently be circumvented by providing the dietary fiber-containing ingredients in the form of comminuted dietary fiber-containing vegetable particles which are incorporated into the composition as defined herein whereby the swelling in the oral cavity is essentially prevented.

As it has been mentioned above, the composition according to the invention is in the form of particles comprising a continuous coating layer containing the amino acid containing ingredient, surrounding an inner core to provide two-component particles in the form of a free-flowing powder. One advantageous embodiment of the invention may be provided by coating as the inner core, particles of a dietary fiber-containing ingredient with the continuous coating layer.

Suitable dietary fiber-containing substance sources include citrus pulp, beet pulp, potato pulp, fruit peel and apple pomace. These sources may have been subjected to various treatments to increase the content of water soluble dietary fiber. Such treatments include as examples treatments with an acid or an alkaline substance. Other useful dietary fiber-containing ingredients may be selected from leguminous seed fibers including pea fiber and soy bean fiber, plant root or tuber fiber products such as potato fiber and cereal fiber products including as examples, oat and wheat brans.

Furthermore, readily water soluble dietary fiber substances such as carrageenans, vegetable and microbially derived gums including Psyllium seed mucilages, guar gum, gum arabicum, xanthan gum, locust bean gum, and alginates may be used in accordance with the present invention. Accordingly, in one preferred embodiment of the invention the iron-containing composition as defined herein comprises a coating layer surrounding a dietary fiber-containing product comprising 20 wt% dried potato pulp, 54 wt% dried apple pomace, 5 wt% dried citrus pulp, 10 wt% guar gum, 10 wt% Psyllium seeds and 1 wt% betaine hydrochloride.

The content of a dietary fiber-containing ingredient in a composition according the present invention is suitably in the range of 1 to 50 wt% such as in the range of 5 to 30 wt%. In other useful embodiments the inner core being coated as defined above may comprise a mixture of a dietary fiber-containing ingredient and the iron compound.

The weight ratio between the coating layer of the present composition and the inner core which it surrounds may be in the range of 10:90 to 99:1. In preferred embodiments, the weight ratio is in the range of 20:80 to 60:40 such as about 1:1. When the composition is one which is in the form of a continuous coating layer surrounding an inner core, the above-mentioned further ingredients may be mixed into the coating layer or they may entirely or partially constitute the inner core.

The administration of a composition as defined herein which is in the form a coating layer surrounding an inner core comprising dietary fiber-containing ingredient may reduce the mortality in suckling animals significantly. It has thus been demonstrated that the mortality rate in suckling piglets to which about 35 g of the composition is administered over a 2 week post partum period may be reduced by about 20% as compared to piglets given an injection of an iron preparation.

An interesting feature of the present composition is the possibility of incorporating herein a relatively high amount of an iron compound having a high content of iron and still obtain a composition which may be ingested voluntarily even by suckling animals. Accordingly, the composition as defined herein is one which when administered as the sole iron source in an amount of at the most 35 g to a piglet during the first two weeks after birth results in a concentration of haemoglobin in the blood of said piglet which is at least 80 g per l, such as at least 90 g per l. Experiments have shown that it is even possible to achieve a concentration of haemoglobin which is at least 100 g per l such as at least 110 g per l.

In one preferred embodiment, a composition as defined herein is provided as a free-flowing powder comprising particles having an average size which is in the range of 10 to 500 µm. In an other preferred embodiment the composition is provided in a form where a plurality of such particles form agglomerates. This is obtained by including in the manufacturing process an agglomeration step. In order to achieve the desired agglomeration of the particles it is required that these particles are only dried to an extent where the outer surface has a sticky consistency allowing the agglomeration to occur. In accordance with the invention, the agglomerates may preferably have an average size which is in the range of 20 µm to 1000 µm, such as in the range of 30 µm to 750 µm.

As it will be explained in details below the present product is obtained by the above-defined spray-drying method of producing a free-flowing powdered iron-containing composition in the form of particles of an inner core carrier material coated with a continuous layer containing the amino acid-containing ingredient.

It may be advantageous to provide the coated iron-containing powder according to the method in the form of agglomerates of the thus coated particles. Accordingly, the coating method may in a preferred embodiment be one wherein only partial solidification of the continuous coating layer in the spray-drying chamber is performed, so that the particles with the partially solidified coating will be moderately sticky so that they will tend to form loose agglomerates when contacting each other, and the moderately sticky particles are collected on a bed of an air-penetrable material in the form of loose agglomerates and are further dried on the bed to substantially completely dry the coating layer on the agglomerated particles.

It is a characteristic feature of most aspects of the production method of the invention that on the one hand the flow of the transport gas with the particles of the carrier material dispersed therein and on the other hand the flow of the drying gas are directed substantially parallel to each other and are regulated so that they form a substantially distinct interface of a substantially constant shape in a region upstream of and adjacent to the region where the collision between the liquid droplets and the particles takes place, and normally, the flow of the transport gas with the carrier particles dispersed therein and the flow of the drying gas are regulated so that the substantially distinct interface of a substantially constant shape prevails also in the region where the collision between the liquid droplets and the particles takes place and in some cases also downstream of the region where the collision between the liquid droplets and the particles takes place.

The distinct interface being of substantially constant shape (in this context, "constant" means constant over time; there may be variations in e.g. cross section shape or dimensions along the path from inlet formation of the interface and downstream) may be assessed by any suitable means, e.g., simply visually.

The flow of the drying gas and the flow of the transport gas are normally regulated so that they are both substantially laminar until the particles have been coated with the coating composition.

In particular suitable embodiments, the flow of the transport gas with the carrier particles dispersed therein is conducted in a substantially annular cross-sectional shape around the atomizing means, such as illustrated in the drawings and discussed in greater detail in the following. In particular, the flow of the transport gas with the carrier particles dispersed therein is conducted in a substantially circular cross-sectional shape around the atomizing means, the atomizing means being arranged substantially centrally in the circle.

While the method of the invention may be performed in such a manner that the particles will be substantially dry while air-borne in the spray drying chamber, in which case they can be removed from the spray drying chamber by, e.g., suction and be carried to a cyclone, an important embodiment of the method is one wherein only partial solidification of the substantially continuous coating layer in the spray-drying or spray-cooling chamber is performed, so that the particles with the partially solidified coating will be moderately sticky so that they will tend to form loose agglomerates when contacting each other, and the moderately sticky particles are collected on a bed of an air-penetrable material in the form of loose agglomerates and are further dried on the bed to substantially completely dry the coating layer on the agglomerated particles. Such a bed of air-penetrable material may be a moving bed of a filter cloth, such as in a "Filtermat" plant as described in the following.

It will be understood that the term "spray drying" as herein may designate both a drying by evaporation and a solidification of a molten coating composition by cooling. Thus, the drying gas is either a gas which has a temperature above the temperature of the transport gas, thus substantially drying the coating composition by supplying heat thereto, or a gas which has such a temperature below the temperature of the transport gas that it will result in solidification of the coating composition by cooling.

As will be discussed in greater detail in the following, an important feature of the production method according to the present invention is that it can be effectively used for coating carrier particles which are of a material which is soluble or swellable in water including as typical examples dietary fiber-containing ingredients.

The discoveries leading to the present invention were arrived at in connection with a thorough investigation of the coating process carried out with a model system consisting of an intensely red coating agent and an almost white powder. By this method, the quality of the coating could be followed directly through microscopy of the final products.

The investigations were concentrated around coating in connection with spray drying. The white powder to be coated consisted of pectin fibres, which swell so quickly upon contact with water that dispersing it in the coating agent was impossible. The coating agent consisted of a red-coloured protein solution, droplets of which are known to shell-dry extremely rapidly.

The investigations showed that even with a small amount of coating composition relative to the powder, a complete coating could be obtained, i.e., no visible white particles could be detected in the red final product prepared according to the invention.

It is believed that the coating composition through its high velocity away from the spraying device displaces a large part of the air molecules in an annular region around the device. Thereby, when the measure according to the present invention are not observed, a vacuum is created which sucks in the drying air. Through the collisions between the coating droplets and the molecules of the drying air, a gradual deceleration of the former takes place with a simultaneous filling of the vacuum. The energy exchange between the hot drying air and the droplets is very intensive in this phase, which results in an almost explosive emission of water molecules from the surface of the droplets. Thereby, the ability of the droplets to spread out on the particle surface quickly becomes diminished which is particularly apparent in operations where a thin layer of coating agent is to be spread over all particles.

The method of the invention solves this problem. It is believed that the powder, dispersed in air, is introduced into the innermost part of the above-mentioned vacuum region, so that the collisions between particles and droplets takes place in an annular region so close around the spraying device that the drying air has not, or only to a small extent, penetrated. Thereby, the dispersion is involved in the atomization process itself, in that the coating agent is flung away from the spraying device in the form of a film which, i.a., through the collisions with gas, such as air, molecules and particles of the dispersion, is split up into fine droplets.

The gas, such as air, used for dispersing the powder should be controlled both with respect to amount and temperature. The amount should be so small that the above-mentioned vacuum region is not filled out. If this happens, white particles will immediately appear in the final product. The temperature should be so low that the properties of the droplets with respect to coating do not deteriorate appreciably.

In the method of the invention, however, the introduction of the dispersion occurs in such a manner that the particles and the droplets form the above-mentioned characteristic flow pattern as long as there is a vacuum in the annular collision region. The part of the flow pattern taken up by the particles are shaped as a sharply defined cylinder, whereas the path taken up by the droplets are shaped like a cone in the case of nozzle spraying and like a disc in the case of centrifugal spraying. The transition between the cylinder and cone/disc is likewise sharply defined. If the supply of dispersion is pushed too high, this transition region starts to become fuzzy and dusty, and in the model system, one will find white particles in the otherwise red product as an indication of incomplete coating.

In coating operations where the coating agent is transferred from a liquid to a solid state through spray cooling, the same principles apply with respect to control of the powder-to-air ratio and of the ratio between the powder dispersion and the coating agent as applies when coating by means of spray drying. On the other hand, the temperature of the air used for dispersing the powder should in this case be so warm that the coating agent does not begin to solidify in the collision region.

Naturally, complete coating requires that there is a sufficient amount of coating agent available to cover the total particle surface. If this is not the case, a small deficiency of coating agent results in a mixture of coated and agglomerated particles, whereas with a larger deficiency of coating agent, a purely agglomerated product is obtained.

The method of the invention has thus proved also to be an effective production method for agglomerated products consisting of partly powdery and partly liquid starting materials.

The supply of an even flow of particles to the collision region may be brought about in different ways depending on whether the coating is to be carried out on spray plants having atomizing wheels or having nozzles.

The supply is most easily solved in spray plants with atomizing wheels, since a preferred embodiment of the supply means consists of a jacket around the conical or cylindrical atomizer housing in suitable distance therefrom, so that the flow of particles dispersed in air is able to pass in the resulting interspace. In a preferred embodiment, the particles are blown tangentially in at the top of the interspace, and the particles will therefore move downward along helical paths to the annular exit opening immediately above the annular collision region. In the preferred embodiment, the tangential inlet is placed in such a manner that the particle flow at the outlet rotates in the opposite direction of the atomizing wheel. In this preferred embodiment, partly a completely uniform supply to the entire annular collision region and partly a maximum relative velocity between particles and droplets at the moment of collision is obtained.

As mentioned above, the advantage of this invention is that evaporation of the coating material by means of the hot air is prevented before the collision between the article to be coated and the coating material.

In comparison with the known techniques of particle coating the presently claimed method of producing a coated particle product involves a number of significant advantages. These advantages include the following:
(i) commercially available spray-drying plants can be used,
(ii) the present invention comprises a continuous process which compared to traditional coating methods gives lower production expenses,
(iii) the method of the present invention has the advantage on the one hand that the flow of the transport gas with the particles of the carrier material dispersed therein and on the other hand the flow of the drying gas are directed substantially parallel to each other and are regulated so that they form a substantially distinct interface of a substantially constant shape in a region upstream of and adjacent to and also prevails in the region where the collision between the liquid droplets and the particles takes place. When comparing with the known art, e.g. EP 423701, the present invention, in a simple and efficient manner, provides the separation between the coating composition and the droplets of coating composition by regulation of the air-flows, which is an efficient and flexible regulation resulting in a much more efficient and controlled process and total flexibility with respect to the use of conventional spray drying plants,
(iv) the present invention makes it possible to coat particles which are water-soluble and have irregular shapes.

A special embodiment of the method of the invention can be characterized as a method for coating powder particles in a spray drying or spray cooling plant with a liquid coating agent, said method comprising
(i) dispersing the particles uniformly in an air flow with controlled ratio between powder and air,
(ii) conducting the dispersion of powder and air into contact with the coating agent at the innermost part of the annular vacuum region formed through the movement of the coating agent droplets away from the atomizing means and to which region the drying or cooling air is prevented from penetrating,
(iii) controlling the amount of the dispersion of powder in air supplied per unit of time in such a manner that it is at any time smaller than the amount required to fill the annular vacuum region,
(iv) the control of the ratio between the amount of the dispersion of powder in air and the coating agent being exerted on the basis of visual or instrument recording or the characteristic flow pattern formed by the powder particles immediately prior to and subsequent to the collision with the coating agent droplets, and
(v)controlling the temperature of the dispersing air flow independent of the drying or cooling air so as to delay the initial transition of the liquid coating agent into solid form till after the collision with the powder particles.

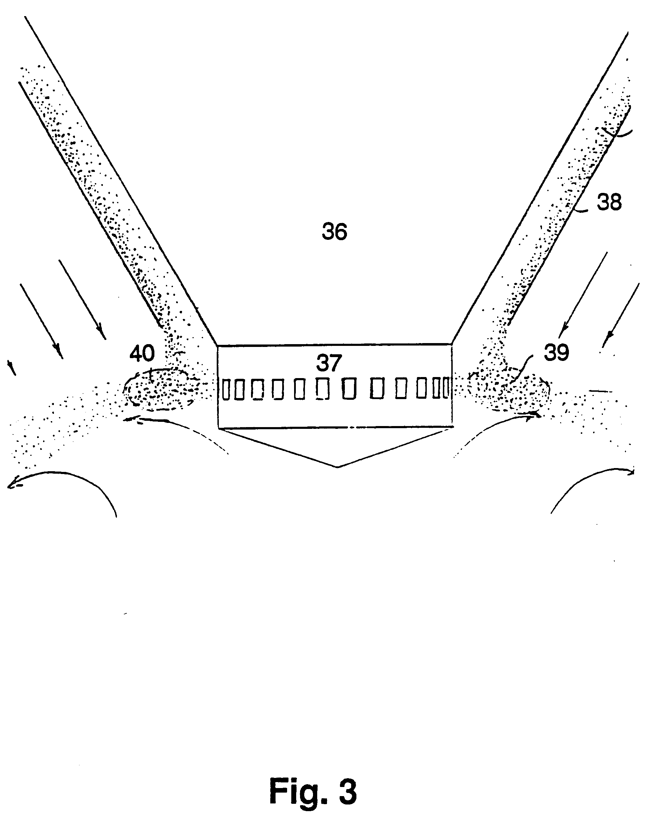

Fig. 3 shows coating in connection with a spray plant having a conical atomizer housing 36 and an atomizing wheel 37. A jacket 38 is placed at a distance from the atomizing wheel, which ensures a suitable air and particle velocity in the interspace between the atomizer housing and the jacket. Reference numerals 39 and 40 designate the annular collision region.

In plants with nozzle atomization, which normally have several nozzles, the supply means to the individual nozzle consists in the preferred embodiment of a double walled tube where the nozzle and its feed tube are placed at the centre, and where the particles are blown in tangentially into the interspace between the walls the end opposite the nozzle.

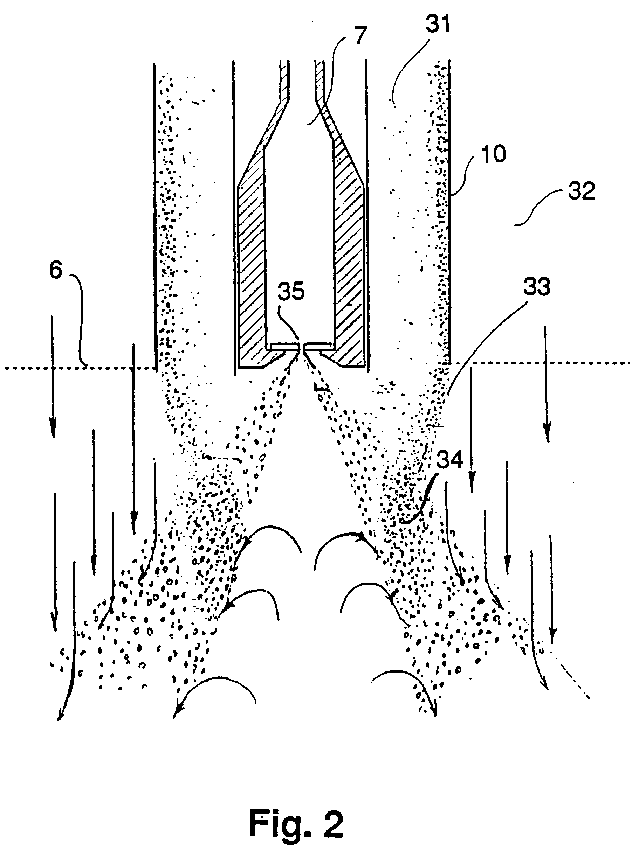

Fig. 2 shows coating in connection with a spray plant with nozzle atomization. A nozzle 7 with a perforated disc 35 atomizes the coating agent so that the droplets move away from the disc 35 in paths describing a hollow cone. A double walled tube defines an interspace 31 conducting the particle flow to the annular collision region 34.

In the event of several nozzles, however, this means that the adjusted flow of particles to the spray plant must be divided into a number of equal partial flows to each respective nozzle. In a preferred embodiment, this is done by supplying the adjusted total particle flow into the centre of a centrifugal ventilator which has as many exits as there are nozzles. By giving the exits completely identical shapes, it is ensured that the partial flows of particles to the individual nozzles will be exactly the same.

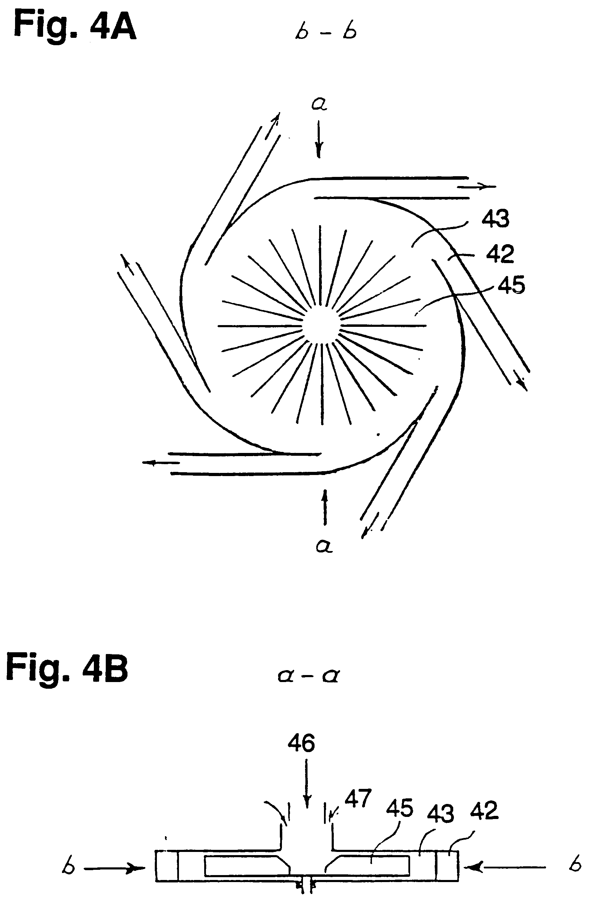

Fig. 4 shows a centrifugal ventilator with 6 exits for dividing a flow of powder into 6 partial flows. Reference numeral 43 designates a ventilator housing, and reference numeral 42 designates one of six identical exits which are arranged in a rotation-symmetrical manner. Reference numeral 45 designates the ventilator wheel, reference numeral 46 designates the central inlet for the powder flow, and reference numeral 47 designates the inlet for the transport air.

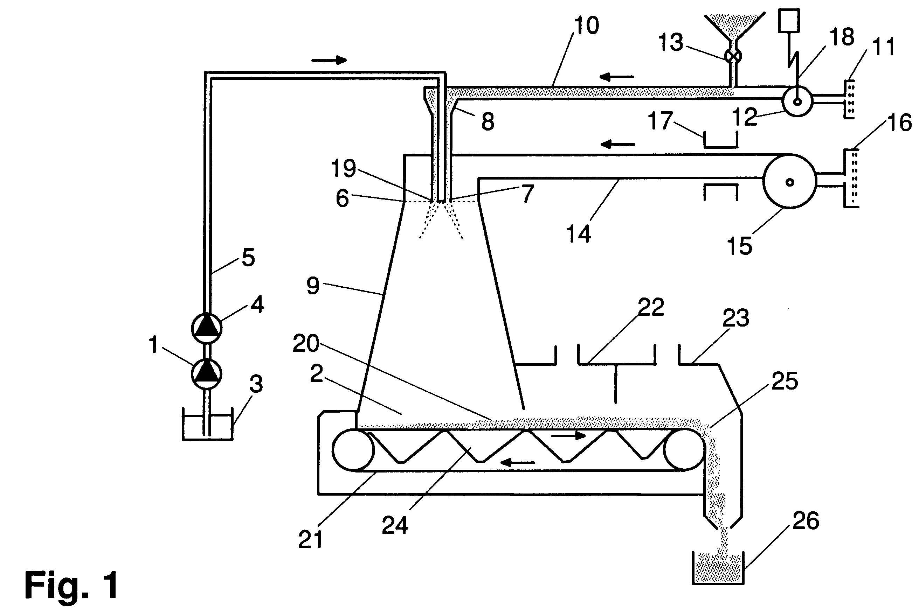

Fig. 1 diagrammatically illustrates what may be considered as a conventional filtermat spray-drying or spray-cooling plant. This kind of spray-drying or spray-cooling plant may preferably be used when carrying out the method according to the invention. The spray-drying or spray-cooling plant comprises a spray-drying or spray-cooling chamber 9 wherein a carrier material is coated with a coating composition. The spray-drying or spray-cooling plant illustrated in Fig. 1 is provided with nozzle means for atomizing a liquid coating composition in a liquid form. However, a spray-drying or spray-cooling plant having an atomizing wheel for atomizing the liquid coating composition may also be used. The spray-drying or spray-cooling plant in Fig. 1 could be provided with a number of nozzles, such as 1-24, but to simplify the description the spray-drying or spray-cooling plant in Fig. 1 is only provided with one nozzle.

A coating composition in liquid form is introduced into the spray-drying or spray-cooling chamber 9 through a high-pressure atomizing nozzle 7 (positioned in the upper part, preferably 6-8 m, more preferably 7 m above the bottom of the of the spray-drying or spray-cooling chamber 9) atomizing the liquid coating composition into droplets. The high-pressure atomizing nozzle 7 is fed with the liquid coating composition under pressure via a supply pipe 5. The pressure is generated by a high-pressure feed pump 4 fed by a feeding pump 1 and communicating with a storage 3 containing the liquid coating composition. The spray-drying or spray-cooling chamber 9 has the form of a hollow frustum of a pyramid, with an opening 2 in the bottom, this opening 2 is preferably of 2.2 m and an upper almost circular opening 8, preferably having an inner diameter in the order of 1.2 m. The high-pressure feed pump 4 generating a pressure in the range of 50-400 atm, preferably in the order of 200 atm. The feeding pump 1, preferably generating a pressure in the range of 2-3 atm. The supply pipe 5 is a high-pressure pipe, preferably with an inner diameter in the order of 8 mm.

The particles of the inner core carrier material, to which the droplets of liquid coating composition is applied, are supplied with a flow of a transport gas, preferably air, to the spray-drying or spray-cooling chamber 9 from an inlet 19 communicating with a particle transporting pipe 10. The particles of the carrier material are dispensed from a dosing means 13 into the particle-transporting pipe 10 and air-borne by the flow of transport gas to the inlet 19. The flow of the transport gas is derived by a fan 12 from the atmosphere through an inlet air filter 11.

After coating of the particles in the spray-drying or spray-cooling chamber 9, the air-borne coated particles are brought into contact with a drying gas, preferably air, in order to solidify the coating at least partly. The drying air is through a grid 6 supplied from a supply pipe 14 communicating with a fan 15 deriving air from the atmosphere through an inlet air filter 16. The grid provides a minor pressure drop in the drying air and give a more laminar flow of the drying air into the spray-drying chamber 9. Depending on the actual process to be carried out by the spray-drying plant, the supply pipe 14 may be provided with air-heating means, such as a gas burner 17.

The direction and the rate of flow of the transport gas are adapted by means of a regulator 18, preferably a frequency converter, regulating the flow through the fan 12. This adaptation substantially prevents contact between, on the one hand, the drying gas and, on the other hand, the droplets so that the liquid coating composition, before any substantial drying thereof, will form a substantially continuous-liquid coating layer on the particles.

Normally, the supply of drying gas or cooling gas may be adjusted by regulators not shown in the drawings.

The particles with at least partly solidified coating in the spray-drying or spray-cooling chamber 9 will be moderately sticky so that they will tend to form loose agglomerates 20 when contacting each other on a movable filtermat belt 21 of air-penetrable material in the form of loose agglomerates. The velocity of the filtermat belt 21 is preferably adjustable. The agglomerates 20 are transported by the belt 21 into at least one drying chamber 22 performing a further second drying of the agglomerates 20 and preferably into a cooling chamber 23 for cooling of the agglomerates 20 before leaving the movable filtermat belt 21. The finished agglomerates 20 are transported to a point 25 where the belt returns, the agglomerate here fall into a hopper 26 for optional further processing. The belt may preferably have a length of 10-12 m, and preferably a width of 1.5-2.0 m such as 1.8 m.

Under the movable filtermat belt 21 in positions under the spray-drying or spray-cooling chamber 9, the chamber 22 and the chamber 23, respectively, exhaust chamber 24 connected to adjustable exhausting fans are provided. The exhaust chamber 24 draws the drying or cooling air through the mat of product and the air-penetrable material. The air-penetrable material is preferably of polyester or polypropylene and is preferably of a kind of fabric having small loops in its outer weaver and bigger loops in its inner weaver so as to prevent material from getting stocked in the fabric.

The high-pressure atomizing nozzle 7 used in the spray-drying and spray-cooling plant illustrated in Fig. 1 is preferably a whirl chamber nozzle providing a mist having a hollow cone shape.

The movable filtermat belt 21, the drying chamber 22, the cooling chamber 23, the exhaust chamber 24 and fans 12 and 15 may preferably be parts from a F500 NIRO Filtermat plant.

Fig. 2 illustrates the zone around a high-pressure atomizing nozzle 7 operating in a spray-drying or spray-cooling plant as described in Fig. 1. In this zone, on the one hand, a flow of a transport gas 31 with particles of a carrier material dispersed therein (supplied from a particle-transporting pipe 10) and, on the other hand, a flow of the drying or cooling gas 32 (supplied from a supply pipe 14) are directed substantially parallel to each other and are regulated so that they form a substantially distinct interface of a substantially constant shape in a region 33 upstream of and adjacent to a region 34 in which the collision between the dispensed liquid droplets and the particles takes place. The droplets are dispensed from the high-pressure atomizing nozzle 7 having a disc 35 with a central opening wherefrom the droplets are dispensed in a hollow conical mist.
- 1: feeding pump
- 2: opening
- 3: storage
- 4: high-pressure feed pump
- 5: supply pipe
- 6: grid
- 7: high-pressure atomizing nozzle
- 8: cyclone
- 9: spray-drying or spray-cooling chamber
- 10: particle-transporting pipe
- 11: inlet air filter
- 12: fan
- 13: dosing means for carrier material
- 14: supply pipe
- 15: fan
- 16: inlet air filter
- 17: air-heating means or air-cooling means
- 18: regulator
- 19: inlet for transport gas
- 20: agglomerated coated particles
- 21: movable filtermat belt
- 22: secondary drying or cooling chamber
- 23: cooling chamber
- 24: exhaust chamber
- 25: product release point
- 26: hopper
- ....:
- 31: transport gas
- 32: drying or cooling gas
- 33: collision region
- 34: collision region
- 35: disc
- ....:
- 36: atomizer housing
- 37: atomizing wheel
- 38: jacket
- 39: collision region
- 40: collision region
- ....:
- 42: exit
- 43: ventilator housing
- 45: ventilator wheel
- 46: powder inlet
- 47: transport gas inlet

As mentioned above, the present invention relates in a still further aspect to a method of dietetically supplying iron to an individual suffering from anaemia, comprising administering to the individual a composition as defined herein. The composition is administered in an amount and for a period of time which results in the maintenance of a physiologically acceptable concentration of haemoglobin in the blood. Preferably, the concentration of haemoglobin to be obtained and maintained is at least 90 g per l of blood. The method is particularly feasible for the prevention of anaemia in suckling piglets. A suitable iron supplementation regime in suckling piglets is the administration of the present composition to the piglets over a period of two weeks post partum in a total amount of at the most 35 g per piglet. The composition is e.g. administered by scattering it on the floor of the farrowing pen from where it is voluntarily ingested by the piglets. One convenient regime of administration of the composition is to divide the total dosage into six or seven dosages which is administered every second day during the period of administration.

It will, however, be understood that the method as defined herein is not limited to use in suckling piglets. Other animals, especially animals on a predominant diet of milk or milk replacer may occasionally have a dietary intake of iron which is not sufficient to secure a physiologically acceptable haemoglobin level. The feeding of the present iron-containing composition may constitute a convenient method of providing an adequate iron supplementation to such animals. The composition may be administered to these animals by incorporating a suitable amount hereof in the milk or milk replacer diet or it can be offered to the animals as a separate feed additive. Furthermore, the method may be used to repair an iron deficiency in humans suffering from anaemia.

The invention is further illustrated in the below Examples:

### EXAMPLE 1

### Preparation of an iron-containing composition

A liquid mixture comprising the following ingredients was prepared:

| | Amount | % dry matter |
|---|---|---|
| Hydrolysed haemoglobin | 325.0 kg | 28 |
| Alcalase™ Food Grade¹⁾ | 0.46 kg | 100 |
| NaOH | 9.9 kg | 27 |
| Rendered lard | 132.0 kg | 100 |
| Panodan™²⁾ | 1.32 kg | 100 |
| Ferrous fumarate | 511.0 kg | 27 |
| E vitamin | 0.05 kg | 100 |
| Lysine | 0.82 kg | 100 |
| Methionine | 2.09 kg | 100 |
| Threonine | 1.23 kg | 100 |
| Tryptophane | 0.41 kg | 100 |
| Total weight | 983.78 kg | |
| Dry matter | 369.84 kg | 37.6 |

| | | |
|---|---|---|
| 1) Novo Nordisk, containing Subtilisin Carlsberg | | |
| 2) A commercial emulsifying agent (Grindsted Products) | | |

The hydrolysed haemoglobin was prepared essentially as described in Olsen, Zeitschrift für Lebensmittel - Technologie und Verfahrenstechnik, 1983, vol. 34, issue 5 on the basis of frozen blood cells separated from porcine blood collected under substantially sterile conditions in a slaughterhouse, by centrifugation followed by freezing. The frozen blood cells were thawed by circulation over a heat exchanger to obtain a thawed blood cell suspension having a temperature of about 55°C which was subsequently subjected to the hydrolysis treatment as follows:

The Alcalase™ Food Grade was added directly to the thawed blood cells in a vessel provided with heating and agitating means and the NaOH were added to the mixture by means of a dosing pump controlled by a pH meter to obtain and maintain a pH in the range of 8.0 to 8.5. The temperature in the reaction mixture was about 55°C and the reaction time was about 4 hours until a degree of hydrolysis (DH) of 18-20 was obtained.

The ferrous fumarate was derived from a freshly prepared batch prepared substantially in accordance with the method described in US 3,478,073. Initially, 375 kg of dry NaOH in pellets were dissolved under agitation by means of a centrifugal pump in 1200 kg of boiled out water in a 6 m³ tank. 2 x 500 kg of ferrous sulphate (FeSO₄,7H₂O) was dissolved in 2 x 1200 kg of boiled water in a 2 m³ tank and subsequently pumped through a filtration bag by means of a centrifugal pump into the tank containing the NaOH solution to obtain the formation of ferrous hydroxide and sodium sulphate. 450 kg of fumaric acid was added slowly to the 6 m³ tank during agitation by means of a centrifugal pump to obtain a suspension of precipitated ferrous fumarate. This suspended ferrous fumarate precipitate was pumped through a filter to remove particles having a size of more than about 50 µm followed by a decanting step whereby most of the sodium sulphate solution was removed.

The resulting ferrous fumarate slurry containing 30 to 35 wt% of the salt was transferred to a 2 m³ tank and subjected to further decanting followed by returning the resulting slurry to the 6 m³ tank. About 2 m³ of boiled water was added while agitating by means of the centrifugal pump. The decanting step was repeated twice and 511 kg of the thus washed ferrous fumarate slurry having a dry matter content of about 27 wt% was used in the further processing.

An emulsion of the rendered lard (food grade) and the hydrolysed haemoglobin was prepared using the Panodan™ emulsifying agent, by pumping the hydrolysed haemoglobin at a temperature in the range of 45 to 50°C into a mixing vessel following by pumping the lard, also at a temperature in the range of 45 to 50°C, into the vessel under vigorous agitation, however, without whisking in air. The mixture was emulsified by means of a Greaves™ mixturing equipment for about 10 minutes followed the addition under vigorous agitation of the ferrous fumarate, the E vitamin and the amino acids and the resulting liquid emulsified mixture was transferred to the feeding tank of a high pressure pump.

In a subsequent coating step the resulting liquid iron-containing composition was applied in a spray-drying coating process to the surface of the particles of a dry powdered mixture of the following ingredients, prepared by blending in a Nauta™ blending apparatus:

| | |
|---|---|
| Biopect™³⁾ | 82.0 kg |
| Vitamin mixture | 9.0 kg |

| | |
|---|---|
| 3) Biopect™ is a powdered pectin-containing product consisting of the following ingredients: 20 wt% of dried potato pulp, 54 wt% of dried apple pomace, 5 wt% of citrus pulp, 10 wt% of carrageenan, 10 wt% of Psyllium seeds and 1 wt% of betaine hydrochloride. The product particles have an average size of about 50 µm (in the range of 10 µm to 100 µm) | |

A coating step was then carried out in a spray-drying plant in the following manner using a spray-drying equipment of the type Filtermat™ F500 from Damrow with an evaporation capacity of 500 lbs/hour. This spray-drying equipment comprises a spray drying chamber and an atomizing means in the form of a spray nozzle of type Delavan™ working at a pressure of 150 bar and giving an open hollow cone spray.

The Filtermat™ F500 equipment consisted of the following components:
(i) a spray-drying tower with a square bottom of 2 x 2 m, the distance from the spray nozzle to the conveyor belt being 7 m,
(ii) an application pipe for the particles of the carrier material having a diameter of 12 cm, the pipe being equipped in the centre with an internal pipe having an internal diameter of 8 mm which internal pipe ends in the nozzle,
(iii) a Filtermat belt with a width of 1.8 m and a total length of 11 m, the part of the belt receiving the coated powder being 5 m. The Filtermat belt which comprises an air-penetrable filter cloth made of a woven 2-layer polymeric material and woven in a way so that the air-penetrating pores of the upper layer have a smaller diameter than the pores of the bottom layer,
(iv) a retention chamber over the Filtermat belt next to the spray tower,
(v) a secondary drying chamber next to the retention chamber and
(vi) a cooling chamber at the outlet end of the belt.

The drying air in the spray tower had an inlet temperature of about 250°C and an outlet temperature of about 75°C.

The coating step comprised supplying as a coating composition the above liquid iron salt-containing emulsion to the spray nozzle of the spray-drying equipment and atomizing the liquid coating composition into a flow of droplets followed, supplying a flow of transport gas comprising as a carrier material particles of the above powdered pectin-containing mixture dispersed therein to the spray-drying chamber separately from the coating composition, supplying a flow of drying gas to the chamber at a temperature which tended to solidify the liquid coating composition.

During operation, the equipment was regulated so as to allow droplets in the flow of liquid droplets of the coating composition to collide with the particles of the carrier material dispersed in the transport gas, the direction and rate of flow of the transport gas being adapted so that contact between on one hand the drying gas and on the other hand the droplets was substantially prevented whereby the liquid coating composition, before any substantial drying thereof, formed a substantially continuous liquid layer on the carrier material particles. Subsequently, the thus applied continuous coating layer on the particles were allowed to partially dry by contact with the drying gas.

When discharged from the spray tower the coated particles were further dried in the retention chamber and in the drying chamber of the filter bed by means of air having an inlet temperature of about 75°C and an outlet temperature of about 60°C. The dried and coated particles were further cooled at ambient temperature.

From the above coating process there was obtained an iron-containing composition in the form of a free-flowing powder comprising agglomerates of primary particles having an average size of about 50 µm in which agglomerates the primary particles were in the form of a continuous coating layer consisting of the dry matter of the above iron salt-containing emulsion surrounding an inner core of the pectin-containing composition. The resulting composition had a content of elemental ferrous iron of about 9.6 wt% and a content of the pectin-containing composition of about 18 wt%. The weight ratio between the amount of coating layer dry matter and the inner core composition was about 4:1.

### EXAMPLE 2

### The effect of an iron-containing composition on blood composition and mortality rate in piglets

A one-year trial including 950 litters of piglets was carried out under the supervision of the Landsudvalget for Svin (Danish National Committee for Pig Breeding). Half of the litters was offered about 60 g of a product as defined in Example 1 per litter on mondays, wednesdays and fridays during the first two weeks of life. This dosage corresponded to about 33 per pig during the two week test period. The other half of the litters (control) were given 200 mg of iron by an injection on the third day of life of a commercial iron-containing injectable preparation.

The test piglets were willing to ingest all of the composition. At the end of the second and the third week, respectively, blood samples of all piglets were collected and the samples analyzed for the concentration of haemoglobin (g/l) and for the haematocrit value (the percentage of the blood that is cells).

The results af the trial are summarized in Table 1 below:

**Table 1**

| Concentration of haemoglobin ((g/l) and haematocrit (per cent) | | | | |
|---|---|---|---|---|
| Age Group | 2 weeks | | 3 weeks | |
| | Injection | Composition | Injection | Composition |
| No. of samples | 80 | 80 | 80 | 80 |
| Haemoglobin | 117 | 117 | 129 | 120 |
| Haematocrit | 40 | 40 | 43 | 40 |

The test litters had an average diarrhoeal frequency of 9.2% during the first week of life whereas the control litters had a corresponding frequency of 10.3%. The mortality rate in test litters during the period from the third day of life and until weaning was 3.9 and the corresponding rate for control litters was 4.7 (p < 0.09).

### EXAMPLE 3

### The effect of an iron-containing composition on blood composition and mortality rate in piglets

A trial was carried out over a 3 month period on the commercial pig unit at the Lancashire College of Agriculture and Horticulture. The purpose of the trial was to record the performance of piglets on two different iron supplementation regimes, viz. an injection of 1 ml iron dextran on day 2 of the piglets life (control groups) as compared to an oral supplementation of about 60 grammes of the composition as defined in Example 1 administered as described in Example 2 by scattering the composition on the floor of the littering pen (test groups). A total of 15 litters per treatment were recorded. In total 296 piglets participated in the trial.

The following data were recorded: (1) blood characteristics, measured at both 10 and 21 days of age including haemoglobin concentration, serum iron concentration and haematocrit values and (2) mortality rates.

The results of blood sample analyses (average for all animals) are summarized in Table 2:

**Table 2**

| Blood characteristics (concentration of haemoglobin, g/l, serum iron, µg/100 ml and haematocrit, per cent) | | | | |
|---|---|---|---|---|
| Age Group | 10 days | | 21 days | |
| | Injection | Composition | Injection | Composition |
| Haemoglobin | 100 | 107 | 102 | 118 |
| Serum iron | 80 | 167 | 42 | 98 |
| Haematocrit | 32 | 34 | 32 | 37 |

On day 10 the mortality rate was 6.2% in the control group as compared to only 2.7 in the test group. On day 21 the corresponding figures for the period of day 10 to 21 were 2.9 versus 1.4. For the period of day 0 to 21 the mortality rate was 9.0 in the control group versus 4.0 in the test group. This difference is statistically significant (p < 0.05).

Based on the results of this trial it can also be concluded that the uptake of iron was superior in the piglets offered the composition of the present invention compared to that of the iron injection (control) piglets at both day 10 and day 21 post partum.

## Claims

1. A composition containing gastrointestinally absorbable iron, comprising 0.1 to 25 wt% of elemental iron and 1 to 99 wt% of an amino acid-containing ingredient, the composition being in the form of a free-flowing powder of particles comprising a continuous coating layer containing the amino acid-containing ingredient and an inner core surrounded by the coating layer.

2. A composition according to claim 1 wherein the particles have an average size which is at the most 1 mm.

3. A composition according to claim 1 wherein the elemental iron is provided as an organic iron compound.

4. A composition according to claim 3 wherein the organic iron compound is selected from a fumarate, an acetate, a glutamate, a succinate, a formate, a lactate, a dextran, a propionate, a fatty acid salt of iron, an amino acid salt of iron and a mixture thereof.

5. A composition according to claim 4 wherein the organic iron compound is ferrous fumarate.

6. A composition according to claim 3 wherein the content of iron is in the range of 3 to 10 wt%, calculated on the composition.

7. A composition according to claim 3 wherein the organic iron compound is provided in the form of particles of an average size which is at the most 50 µm.

8. A composition according to claim 7 wherein the organic iron compound particles have an average size which is at the most 30 µm, preferably at the most 10 µm.

9. A composition according to claim 1 wherein the amino acid-containing ingredient is selected from a protein, a peptide, an amino acid and a mixture thereof.

10. A composition according to claim 9 wherein the amino acid-containing ingredient is a protein hydrolysate.

11. A composition according to claim 10 wherein the protein hydrolysate has a degree of hydrolysis which is at least 5%.

12. A composition according to claim 11 wherein the protein hydrolysate has a degree of hydrolysis which is at least 10%.

13. A composition according to claim 12 wherein the protein hydrolysate is derived from haemoglobin.

14. A composition according to claim 1 wherein the content of the amino acid-containing ingredient is in the range of 5 to 30 wt%, calculated on the composition.

15. A composition according to claim 1 wherein the coating layer further comprises a fatty acid-containing substance in an amount which is in the range of 1 to 50 wt%, calculated on the composition.

16. A composition according to claim 15 wherein the amount of the fatty acid-containing substance is in the range of 5 to 20 wt%, calculated on the composition.

17. A composition according to claim 1 wherein the coating layer comprises an emulsified mixture of the amino acid-containing ingredient and a fatty acid-containing substance.

18. A composition according to claim 1 which further contains at least one ingredient selected from a dietary fiber-containing ingredient, a flavouring agent, a vitamin, a micronutrient, a bacterial culture, an enzyme, an alkaline substance and an acid substance.

19. A composition according to claim 1 wherein the inner core being coated comprises a dietary fiber-containing ingredient.

20. A composition according to claim 19 wherein at least 50% of the dietary fiber of the dietary fiber-containing ingredient is soluble in water.

21. A composition according to claim 19 wherein the content of the dietary fiber-containing ingredient is in the range of 1 to 50 wt%, calculated on the composition.

22. A composition according to claim 21 wherein the content of the dietary fiber-containing ingredient is in the range of 5 to 30 wt%, calculated on the composition.

23. A composition according to claim 1 wherein the elemental iron is contained in the coating layer.

24. A composition according to claim 1 or 23 wherein the inner core comprises a mixture of a dietary fiber-containing ingredient and the organic iron compound.

25. A composition according to claim 1 wherein the weight ratio between the coating layer and the inner core is in the range of 10:90 to 99:1.

26. A composition according to claim 25 wherein the weight ratio is in the range of 20:80 to 60:40.

27. A composition according to claim 26 wherein the weight ratio is about 1:1.

28. A composition according to claim 1 which is a free-flowing powder comprising particles having an average size which is in the range of 10 to 500 µm.

29. A composition according to claim 1 wherein a plurality of particles form agglomerates.

30. A composition according to claim 29 wherein the particle agglomerates have an average size which is in the range of 20 to 1000 µm.

31. A composition according to claim 30 wherein the particle agglomerates have an average size which is in the range of 30 to 750 µm.

32. A composition according to claim 1 which, when administered as the sole iron source in an amount of at the most 35 g to a piglet during the first two weeks after birth, results in a concentration of haemoglobin in the blood of said piglet which is at least 80 g per l.

33. A composition according to claim 32 which, when administered as the sole iron source in an amount of at the most 35 g to a piglet during the first two weeks after birth, results in a concentration of haemoglobin in the blood of said piglet which is at least 90 g per l.

34. A method of producing a free-flowing powdered iron-containing composition comprising particles of an inner core carrier material coated with a continuous layer of a coating composition comprising an amino acid-containing ingredient, the method comprising
(i) preparing a liquid coating composition comprising an amino acid-containing ingredient in an amount in the range of 1 to 99 wt%, calculated on the iron-containing composition,
(ii) providing a particulate carrier material having an average particle size being in the range of 1 µm to 100 µm
(iii) adding to the coating composition and/or the inner core carrier material an iron compound in an amount providing a content of elemental iron which is in the range of 0.1 to 25 wt%, calculated on the iron-containing composition,
(iv) supplying the coating composition in liquid form to the atomizing means of a spray-drying plant comprising a spray-drying chamber, and atomizing the liquid coating composition into a flow of droplets,
(v) supplying a flow of transport gas comprising particles of the carrier material dispersed therein to the spray-drying chamber separately from the coating composition,
(vi) supplying a flow of drying gas to the chamber at a temperature which tends to solidify the liquid coating composition,
(vii) allowing droplets in the flow of liquid droplets of the coating composition to collide with the particles of the carrier material dispersed in the transport gas, the direction and rate of flow of the transport gas being adapted to substantially prevent contact between on one hand the drying gas and on the other hand the droplets, so that the liquid coating composition, before any substantial drying thereof, will form a substantially continuous liquid layer on the carrier material particles,
(viii) then allowing the thus applied continuous coating layer on the particles to at least partially dry by contact with the drying gas, and
(ix) withdrawing the coated particles from the spray-drying chamber.

35. A method according to claim 34 wherein on the one hand the flow of the transport gas with the particles of the carrier material dispersed therein and on the other hand the flow of the drying gas are directed substantially parallel to each other and are regulated so that they form a substantially distinct interface of a substantially constant shape in a region upstream and adjacent to the region where the collision between the liquid droplets and the particles takes place.

36. A method according to claim 34 or 35 wherein only partial solidification of the continuous coating layer in the spray-drying chamber is performed, so that the particles with the partially solidified coating will be moderately sticky so that they will tend to form loose agglomerates when contacting each other, and the moderately sticky particles are collected on a bed of an air-penetrable material in the form of loose agglomerates and are further dried on the bed to substantially completely dry the coating layer on the agglomerated particles.

37. A method according to claim 34 wherein the elemental iron is provided as an organic iron compound.

38. A method according to claim 37 wherein the organic iron compound is selected from a fumarate, an acetate, a glutamate, a succinate, a formate, a lactate, a dextran, a propionate, a fatty acid salt of iron, an amino acid salt of iron and a mixture thereof.

39. A method according to claim 34 wherein the elemental iron is provided in the form of organic iron compound particles of an average size which is at the most 50 µm.

40. A method according to claim 39 wherein the organic iron compound particles have an average size which is at the most 30 µm, preferably at the most 10 µm.

41. A method according to claim 34 wherein the amino acid-containing ingredient is selected from a protein, a peptide, an amino acid and a mixture thereof.

42. A method according to claim 41 wherein the amino acid-containing ingredient is a protein hydrolysate.

43. A method according to claim 42 wherein the protein hydrolysate has a degree of hydrolysis which is at least 5%.

44. A method according to claim 43 wherein the protein hydrolysate is derived from haemoglobin.

45. A method according to claim 34 wherein the content of the amino acid-containing ingredient is in the range of 5 to 30 wt%, calculated on the composition.

46. A method according to claim 34 wherein the coating layer further comprises a fatty acid-containing substance in an amount which is in the range of 1 to 50 wt%, calculated on the composition.

47. A method according to claim 46 wherein the amount of the fatty acid-containing substance is in the range of 5 to 20 wt%, calculated on the composition.

48. A method according to claim 34 wherein the coating layer comprises an emulsified mixture of the amino acid-containing ingredient and a fatty acid-containing substance.

49. A method according to claim 34 wherein the inner core being coated comprises a dietary fiber-containing ingredient.

50. A method according to claim 49 wherein at least 50% of the dietary fiber of the dietary fiber-containing ingredient is water soluble.

51. A method according to claim 49 wherein the content of the dietary fiber-containing ingredient inner core material is in the range of 1 to 50 wt%, calculated on the composition.

52. A method according to claim 51 wherein the content of the dietary fiber-containing ingredient is in the range of 5 to 30 wt%, calculated on the composition.

53. A method according to claim 34 wherein the inner core carrier material comprises a mixture of a dietary fiber-containing ingredient and the organic iron compound.

54. A method according to claim 34 wherein the weight ratio between the coating layer and the inner core is in the range of 10:90 to 99:1.

55. A method according to claim 54 wherein the weight ratio is in the range of 20:80 to 60:40.

## Patentansprüche

1. Zusammensetzung, die gastrointestinal absorbierbares Eisen enthält und 0,1 bis 25 Gew.-% elementares Eisen sowie 1 bis 99 Gew.-% eines Aminosäure enthaltenden Bestandteils umfaßt, wobei die Zusammensetzung in Form eines rieselfähigen Pulvers aus Teilchen vorliegt, die eine kontinuierliche Überzugsschicht mit einem Gehalt an dem Aminosäure enthaltenden Bestandteil und einen inneren Kern umfaßt, der von der Überzugsschicht umgeben ist.

2. Zusammensetzung nach Anspruch 1, bei der die Teilchen eine durchschnittliche Größe von höchstens 1 mm aufweisen.

3. Zusammensetzung nach Anspruch 1, bei der das elementare Eisen als eine organische Eisenverbindung bereitgestellt wird.

4. Zusammensetzung nach Anspruch 3, bei der die organische Eisenverbindung aus einem Fumarat, einem Acetat, einem Glutamat, einem Succinat, einem Formiat, einem Lactat, einem Dextran, einem Propionat, einem Fettsäuresalz von Eisen, einem Aminosäuresalz von Eisen und einer Mischung davon ausgewählt ist.

5. Zusammensetzung nach Anspruch 4, bei der die organische Eisenverbindung Eisen(II)-fumarat ist.

6. Zusammensetzung nach Anspruch 3, bei der der Eisengehalt 3 bis 10 Gew.-% auf Basis der Zusammensetzung beträgt.

7. Zusammensetzung nach Anspruch 3, bei der die organische Eisenverbindung in Form von Teilchen mit einer durchschnittlichen Größe von höchstens 50 µm bereitgestellt wird.

8. Zusammensetzung nach Anspruch 7, bei der die Teilchen der organischen Eisenverbindung eine durchschnittliche Größe von höchstens 30 µm, vorzugsweise höchstens 10 µm aufweisen.

9. Zusammensetzung nach Anspruch 1, bei dem der Aminosäure enthaltende Bestandteil aus einem Protein, einem Peptid, einer Aminosäure und einer Mischung davon ausgewählt ist.

10. Zusammensetzung nach Anspruch 9, bei dem der Aminosäure enthaltende Bestandteil ein Proteinhydrolysat ist.

11. Zusammensetzung nach Anspruch 10, bei der das Proteinhydrolysat einen Hydrolysegrad von mindestens 5 % aufweist.

12. Zusammensetzung nach Anspruch 11, bei der das Proteinhydrolysat einen Hydrolysegrad von mindestens 10 % aufweist.

13. Zusammensetzung nach Anspruch 12, bei der das Proteinhydrolysat von Hämoglobin abgeleitet ist.

14. Zusammensetzung nach Anspruch 1, bei der der Gehalt des Aminosäure enthaltenden Bestandteils 5 bis 30 Gew.-% bezogen auf die Zusammensetzung beträgt.

15. Zusammensetzung nach Anspruch 1, bei der die Überzugsschicht weiterhin eine Fettsäure enthaltende Substanz in einer Menge von 1 bis 50 Gew.-% bezogen auf die Zusammensetzung umfaßt.

16. Zusammensetzung nach Anspruch 15, bei der die Menge der Fettsäure enthaltenden Substanz von 5 bis 20 Gew.-% bezogen auf die Zusammensetzung beträgt.

17. Zusammensetzung nach Anspruch 1, bei der die Überzugsschicht eine emulgierte Mischung des Aminosäure enthaltenden Bestandteils und einer Fettsäure enthaltenden Substanz umfaßt.

18. Zusammensetzung nach Anspruch 1, die weiterhin mindestens einen Bestandteil enthält, der aus einem diätetischen Faser enthaltenden Bestandteil, einem Geschmacksmittel, einem Vitamin, einem Mikronährstoff, einer Bakterienkultur, einem Enzym, einer alkalischen und einer sauren Substanz ausgewählt ist.

19. Zusammensetzung nach Anspruch 1, bei der der zu beschichtende innere Kern einen Bestandteil umfaßt, der eine diätetische Faser enthält.

20. Zusammensetzung nach Anspruch 19, bei der mindestens 50 % der diätetischen Faser des diätetische Faser enthaltenden Bestandteils in Wasser löslich ist.

21. Zusammensetzung nach Anspruch 19, bei der der Gehalt des diätetische Faser enthaltenden Bestandteils 1 bis 50 Gew.-% bezogen auf die Zusammensetzung beträgt.

22. Zusammensetzung nach Anspruch 21, bei der der Gehalt des diätetische Faser enthaltenden Bestandteils 5 bis 30 Gew.-% bezogen auf die Zusammensetzung beträgt.

23. Zusammensetzung nach Anspruch 1, bei der das elementare Eisen in der Überzugsschicht enthalten ist.

24. Zusammensetzung nach Anspruch 1 oder 23, bei der der innere Kern eine Mischung eines diätetische Faser enthaltenden Bestandteils und der organischen Eisenverbindung umfaßt.

25. Zusammensetzung nach Anspruch 1, bei der das Gewichtsverhältnis zwischen Überzugsschicht und dem inneren Kern 10:90 bis 99:1 beträgt.

26. Zusammensetzung nach Anspruch 25, bei der das Gewichtsverhältnis 20:80 bis 60:40 beträgt.

27. Zusammensetzung nach Anspruch 26, bei der das Gewichtsverhältnis etwa 1:1 beträgt.

28. Zusammensetzung nach Anspruch 1, die ein rieselfähiges Pulver ist, das Teilchen mit einer mittleren Größe von 10 bis 500 µm umfaßt.

29. Zusammensetzung nach Anspruch 1, bei der eine Vielzahl von Teilchen Agglomerate bilden.

30. Zusammensetzung nach Anspruch 29, bei der die Teilchenagglomerate eine durchschnittliche Größe von 20 bis 1000 µm aufweisen.

31. Zusammensetzung nach Anspruch 30, bei der die Teilchenagglomerate eine durchschnittliche Größe von 30 bis 750 µm aufweisen.

32. Zusammensetzung nach Anspruch 1, die, wenn sie als einzige Eisenquelle einem Ferkel während der ersten 2 Wochen nach der Geburt in einer Menge von höchstens 35 g verabreicht wird, zu einer Konzentration von Hämoglobin im Blut des Ferkels von mindestens 80 g/l führt.

33. Zusammensetzung nach Anspruch 32, die, wenn sie als einzige Eisenquelle einem Ferkel während der ersten 2 Wochen nach der Geburt in einer Menge von höchstens 35 g verabreicht wird, zu einer Konzentration von Hämoglobin im Blut des Ferkels von mindestens 90 g/l führt.

34. Verfahren zur Herstellung einer rieselfähigen, pulverisierten, Eisen enthaltenden Zusammensetzung, die Teilchen aus einem Trägermaterial mit einem inneren Kern umfaßt, der mit einer kontinuierlichen Schicht einer Überzugszusammensetzung beschichtet ist, welche einen Aminosäure enthaltenden Bestandteil umfaßt, bei dem man:
(i) eine flüssige Überzugszusammensetzung herstellt, die einen Aminosäure enthaltenden Bestandteil in einer Menge von 1 bis 99 Gew.-% bezogen auf die Eisen enthaltende Zusammensetzung umfaßt,
(ii) ein teilchenförmiges Trägermaterial mit einer durchschnittlichen Teilchengröße von 1 µm bis 100 µm bereitstellt,
(iii) der Beschichtungszusammensetzung und/oder dem Trägermaterial mit einem inneren Kern eine Eisenverbindung in einer solchen Menge zugibt, daß ein Gehalt an elementaren Eisen von 0,1 bis 25 Gew.-% bezogen auf die Eisen enthaltende Zusammensetzung bereitgestellt wird,
(iv) die Überzugszusammensetzung in flüssiger Form in die Zerstäubungs-Vorrichtung einer Sprühtrocknungsanlage mit einer Sprühtrocknungskammer einspeist und die flüssige Überzugszusammensetzung in einen Fluß von Tröpfchen zerstäubt,
(v) einen Transportgasfluß, der Teilchen von darin dispergiertem Trägermaterial umfaßt, separat von der Überzugszusammensetzung in die Sprühtrocknungskammer einspeist, (vi) einen Trocknungsgasfluß, an einer Temperatur, die zur Verfestigung der flüssigen Überzugszusammensetzung beiträgt, in die Kammer einspeist,
(vii) Tröpfchen im flüssigen Tröpfchenfluß der Überzugszusammensetzung mit den Teilchen des im Transportgas dispergierten Trägermaterials kollidieren läßt, wobei die Richtung und Geschwindigkeit des Flusses des Transportgases so eingestellt wird, daß der Kontakt zwischen einerseits dem Trocknungsgas und andererseits den Tröpfchen im wesentlichen vermieden wird, so daß die flüssige Überzugszusammensetzung vor jeglicher wesentlicher Trocknung eine im wesentlichen kontinuierliche flüssige Schicht auf den Trägermaterialteilchen ausbilden wird,
(viii) dann die so aufgebrachte kontinuierliche Überzugsschicht auf den Teilchen mindestens teilweise durch Kontakt mit dem Trocknungsgas trocknen läßt und
(ix) die beschichteten Teilchen aus der Sprühtrocknungskammer entfernt.

35. Verfahren nach Anspruch 34, bei dem man einerseits den Transportgasfluß mit den darin dispergierten Teilchen des Trägermaterials und andererseits den Trocknungsgasfluß im wesentlichen parallel zueinander leitet und so einstellt, daß sie eine im wesentlichen getrennte Zwischenschicht in im wesentlichen konstanter Form in einem Bereich oberhalb und angrenzend an den Bereich bilden, in dem die Kollision zwischen den flüssigen Tröpfchen und den Teilchen stattfindet.

36. Verfahren nach Anspruch 34 oder 35, bei dem man die kontinuierliche Überzugsschicht in der Sprühtrocknungskammer nur teilweise verfestigen läßt, so daß die Teilchen mit dem teilweise verfestigten Überzug leicht klebrig bleiben und lockere Agglomerate bilden, wenn sie miteinander in Kontakt treten, und die leicht klebrigen Teilchen auf einem Bett eines luftdurchdringbaren Materials in Form lockerer Agglomerate sammelt und weiter auf dem Bett bis zur im wesentlichen vollständigen Trocknung der Überzugsschicht auf den agglomerierten Teilchen getrocknet werden.

37. Verfahren nach Anspruch 34, bei dem man das elementare Eisen als eine organische Eisenverbindung bereitstellt.

38. Verfahren nach Anspruch 37, bei dem die organische Eisenverbindung aus einem Fumarat, einem Acetat, einem Glutamat, einem Succinat, einem Formiat, einem Lactat, einem Dextran, einem Propionat, einem Fettsäuresalz von Eisen, einem Aminosäuresalz von Eisen und einer Mischung davon ausgewählt ist.

39. Verfahren nach Anspruch 34, bei dem man das elementare Eisen in Form von Teilchen der organischen Eisenverbindung mit einer durchschnittlichen Größe von höchstens 50 µm bereitstellt.

40. Verfahren nach Anspruch 39, bei dem die Teilchen mit der organischen Eisenverbindung eine durchschnittliche Größe von höchstens 30 µm, vorzugsweise höchstens 10 µm aufweisen.

41. Verfahren nach Anspruch 34, bei dem der Aminosäure enthaltende Bestandteil aus einem Protein, einem Peptid, einer Aminosäure und einer Mischung davon ausgewählt ist.

42. Verfahren nach Anspruch 41, bei dem der Aminosäure enthaltende Bestandteil ein Proteinhydrolysat ist.

43. Verfahren nach Anspruch 42, bei dem das Proteinhydrolysat einen Hydrolysegrad von mindestens 5 % aufweist.

44. Verfahren nach Anspruch 43, bei dem das Proteinhydrolysat von Hämoglobin abgeleitet ist.

45. Verfahren nach Anspruch 34, bei dem der Gehalt des Aminosäure enthaltenden Bestandteils 5 bis 30 Gew.-% bezogen auf die Zusammensetzung beträgt.

46. Verfahren nach Anspruch 34, bei dem die Überzugsschicht ferner eine Fettsäure enthaltende Substanz in einer Menge von 1 bis 50 Gew.-% bezogen auf die Zusammensetzung umfaßt.

47. Verfahren nach Anspruch 46, bei dem die Menge der Fettsäure enthaltenden Substanz 5 bis 20 Gew.-% bezogen auf die Zusammensetzung beträgt.

48. Verfahren nach Anspruch 34, bei dem die Überzugsschicht eine emulgierte Mischung des Aminosäure enthaltenden Bestandteils und einer Fettsäure enthaltenden Substanz umfaßt.

49. Verfahren nach Anspruch 34, bei dem der zu beschichtende innere Kern einen Bestandteil umfaßt, der eine diätetische Faser enthält.

50. Verfahren nach Anspruch 49, bei dem mindestens 50% der diätetischen Faser des diätetische Faser enthaltenden Bestandteils in Wasser löslich ist.

51. Verfahren nach Anspruch 49, bei dem der Gehalt an diätetische Faser enthaltendem inneren Kernmaterial, 1 bis 50 Gew.-% bezogen auf die Zusammensetzung beträgt.

52. Verfahren nach Anspruch 51, bei dem der Gehalt an dem diätetische Faser enthaltenden Bestandteil 5 bis 30 Gew.-% bezogen auf die Zusammensetzung beträgt.

53. Verfahren nach Anspruch 34, bei dem das innere Kern-Trägermaterial eine Mischung aus einem diätetische Faser enthaltenden Bestandteil und der organischen Eisenverbindung umfaßt.

54. Verfahren nach Anspruch 34, bei dem das Gewichtsverhältnis zwischen Überzugsschicht und dem inneren Kern im Bereich von 10:90 bis 99:1 liegt.

55. Verfahren nach Anspruch 54, bei dem das Gewichtsverhältnis im Bereich von 20:80 bis 60:40 liegt.

## Revendications

1. Une composition contenant du fer absorbable par voie gastro-intestinale, comprenant de 0,1 à 25 % en poids de fer élémentaire et de 1 à 99 % en poids d'un ingrédient à base d'acide aminé, la composition étant sous la forme d'une poudre fluide formée de particules comprenant une couche d'enrobage continue contenant un ingrédient à base d'acide-aminé et un noyau intérieur entouré par la couche d'enrobage.

2. Une composition selon la revendication 1, dans laquelle les particules présentent une dimension moyenne qui est au plus de 1 mm.

3. Une composition selon la revendication 1, dans laquelle le fer élémentaire est apporté sous la forme d'un dérivé organique de fer.

4. Une composition selon la revendication 3, dans laquelle le dérivé organique de fer est choisi parmi les fumarate, acétate, glutamate, succinate, formiate, lactate, dextrane, propionique, sel d'acide gras de fer, ainsi qu'un sel d'acide aminé de fer, ainsi qu'un de leurs mélanges.

5. Une composition selon la revendication 4 , dans laquelle le dérivé organique de fer est le fumarate ferreux.

6. Une composition selon la revendication 3, dans laquelle la teneur en fer est comprise entre 3 et 10 % en poids exprimée par rapport à la composition.

7. Une composition selon la revendication 3, dans laquelle le dérivé organique de fer est apporté sous la forme de particules d'une dimension moyenne qui est au plus de 50 µm.

8. Une composition selon la revendication 7, dans laquelle les particules du composé à base de fer organique présentent une dimension moyenne qui est au plus de 30 µm et de préférence au plus de 10 µm.

9. Une composition selon la revendication 1, dans laquelle l'additif à base d'acide aminé est choisi parmi une protéine, un peptide un acide aminé ou un de leurs mélanges.

10. Une composition selon la revendication 9, dans laquelle l'ingrédient à base d'acide aminé est un hydrolysat de protéine.

11. Une composition selon la revendication 10, dans laquelle l'hydrolysat de protéine présente un faux d'hydrolyse qui est d'au moins 5 %.

12. Une composition selon la revendication 11, dans laquelle l'hydrolysat de protéine présente un taux d'hydrolyse qui est d'au moins 10 %.

13. Une composition selon la revendication 12, dans laquelle l'hydrolysat de protéine dérive de l'hémoglobine.

14. Une composition selon la revendication 1, dans laquelle la teneur en ingrédient à base d'acide aminé est comprise entre 5 et 30 %, en poids exprimée par rapport à la composition.

15. Une composition selon la revendication 1, dans laquelle la couche d'enrobage comprend en outre une substance à base d'acide gras en une proportion qui est comprise entre 1 et 50 % en poids exprimée par rapport à la composition.

16. Une composition selon la revendication 15, dans laquelle la quantité de substance à base d'acide gras est comprise entre 5 et 20 % en poids, exprimée par rapport à la composition.

17. Une composition selon la revendication 1, dans laquelle la couche d'enrobage comprend un mélange émulsifié d'un ingrédient à base d'acide aminé et d'une substance à base d'acide gras.

18. Une composition selon la revendication 1, qui contient en outre au moins un ingrédient choisi parmis l'ingrédient à base de fibre diététique, un agent arômatisant, une vitamine, un micronutriment, une culture bactérienne, un enzyme, une substance alcaline et une substance acide.

19. Une composition selon la revendication 1, dans laquelle le noyau intérieur enrobé comprend un ingrédient à base de fibre diététique.

20. Une composition selon la revendication 19, dans laquelle au moins 50 % de la fibre diététique de l'ingrédient à base de fibre diététique est soluble dans l'eau.

21. Une composition selon la revendication 19, dans laquelle la teneur en ingrédient à base de fibre diététique est de 1 à 50 % en poids, exprimé par rapport à la composition.

22. Une composition selon la revendication 21, dans laquelle la teneur en ingrédient à base de fibre diététique est de 5 à 30 % en poids, exprimé par rapport à la composition.

23. Une composition selon la revendication 1, dans laquelle le fer élémentaire est contenu dans la couche d'enrobage.

24. Une composition selon la revendication 1 ou la revendication 23, dans laquelle le noyau intérieur est formé d'un mélange d'un ingrédient à base de fibre diététique et du dérivé organique de fer.

25. Une composition selon la revendication 1, dans laquelle le rapport pondéral entre la couche d'enrobage et le noyau interne est compris entre 10/90 et 99/1.

26. Une composition selon la revendication 25, dans laquelle le rapport pondéral est compris entre 20/80 et 60/40.

27. Une composition selon la revendication 26, dans laquelle le rapport pondéral est de l'ordre de 1/1.

28. Une composition selon la revendication 1, consistant en une poudre fluide comprenant des particules dont la dimension moyenne est comprise entre 10 et 500 µm.

29. Une composition selon la revendication 1, dans laquelle une pluralité de particules forment des agglomérats.

30. Une composition selon la revendication 29, dans laquelle les agglomérats de particules présentent une dimension moyenne qui est comprise entre 20 et 1000 µm.

31. Une composition selon la revendication 30, dans laquelle les agglomérats de particules présentent une dimension moyenne qui est comprise entre 30 et 750 µm.

32. Une composition selon la revendication 1 qui, lorsqu'elle est administrée en tant que seule source de fer en une quantité d'au plus de 35 g à un porcelet durant les deux premières semaines après la naissance, conduit à une concentration d'hémoglobine dans le sang dudit porcelet d'au moins 80 g/litre.

33. Une composition selon la revendication 32 qui, lorsqu'elle est administrée en tant que seule source de fer en une quantité d'au plus de 35 g à un porcelet durant les deux premières semaines après la naissance, conduit à une concentration d'hémoglobine dans le sang dudit porcelet d'au moins 90 g/litre.

34. Un procédé de production d'une composition contenant du fer pulvérulente fluide et comprenant des particules d'une nature de support d'un noyau interne enrobé d'une couche continue d'une composition d'enrobage comprenant un ingrédient à base d'acide aminé, ce procédé comprenant :
(i) la préparation d'une composition d'enrobage liquide comprenant l'ingrédient à base d'acide aminé en une quantité comprise entre 1 et 99% en poids calculée par rapport à la composition contenant le fer;
(ii) l'utilisation d'une matière de support particulaire présentant une dimension moyenne de particule comprise entre 1 µm et 100 µm;
(iii) l'addition, à la composition d'enrobage et/ou à la matière de support formant le noyau interne, d'un dérivé de fer en une quantité correspondant à une teneur en fer élémentaire comprise entre 0,1 et 25 % en poids calculée par rapport à la composition contenant le fer :
(iv) l'amenée de la composition d'enrobage sous forme liquide à des moyens d'atomisation d'une unité de séchage par pulvérisation comprenant une chambre de séchage par pulvérisation et l'atomisation de la composition d'enrobage liquide en un flux de gouttelettes;
(v) l'amenée d'un courant de gaz de transport comprenant des particules de la matière de support dispersée en son sein à la chambre de séchage par pulvérisation séparément de l'amenée de la composition d'enrobage;
(vi) l'amenée d'un flux de gaz de séchage à ladite chambre à une température qui tend à solidifier la composition d'enrobage liquide.
(vii) l'amenée des gouttelettes du flux de gouttelettes de liquide de la composition d'enrobage à heurter les particules de la matière formant le support dispersée dans le gaz de transport, la direction et le débit du flux du gaz de transport étant conçus de façon à pratiquement empêcher tout contact entre, d'une part. le gaz de séchage, et, d'autre part, les gouttelettes, de sorte que la composition d'enrobage liquide, avant tout séchage notable, forme une couche de liquide pratiquement continue sur les particules de matières de support;
(viii) puis séchage au moins partiel de la couche d'enrobage continue ainsi appliquée sur les particules par contact avec le gaz de séchage ; et
(ix) décharge des particules enrobées de la chambre de séchage par pulvérisation.

35. Un procédé selon la revendication 34, dans lequel, d'une part le flux de gaz de transport contenant les particules de matières de support qui sont dispersées en son sein et, d'autre part, le flux de gaz de séchage, sont dirigés de façon pratiquement parallèle l'un à l'autre et sont réglés de façon à ce qu'ils forment une interface pratiquement distincte présentant une forme pratiquement constante dans une région en amont et adjacente à la région dans laquelle la collision entre les gouttelettes liquides et les particules se produit.

36. Un procédé selon la revendication 34 ou 35, dans lequel seulement une solidification partielle de la couche d'enrobage continue dans la chambre de séchage par pulvérisation est effectuée, de sorte que les particules avec l'enrobage partiellement solidifié soient modérément collantes et n'aient donc pas tendance à former des agglomérats lâches lorsqu'on les met au contact les uns des autres et les particules modérément collantes sont recueillies sur un lit d'une matière pénétrable par l'air sous la forme d'un agglomérat lâche et sont en outre séchées sur le lit jusqu'à ce que la couche d'enrobage portée par les particules agglomérées soit pratiquement séchée.

37. Un procédé selon la revendication 34, dans lequel le fer élémentaire est apporté sous la forme d'un dérivé organique de fer.

38. Un procédé selon la revendication 37, dans lequel le dérivé organique de fer est choisi parmis les fumarate, acétate, glutamate, succinate, formiate, lactate, dextrane, propionate, sel d'acide gras de fer, ainsi qu'un sel d'acide aminé de fer d'acide aminé, ainsi qu'un de leurs mélanges.

39. Un procédé selon la revendication 34, dans lequel le fer élémentaire est amené sous la forme de particules de dérivé organique de fer dont la dimension moyenne est au plus de 50 µm.

40. Un procédé selon la revendication 39, dans lequel les particules dérivé organique de fer présentent une dimension moyenne qui est au plus de 30 µm et de préférence au plus de 10 µm.

41. Un procédé selon la revendication 34, dans lequel l'ingrédient à base d'acide aminé est choisi parmi une protéine, un peptide, un acide aminé ou un de leurs mélanges.

42. Un procédé selon la revendication 41, dans lequel l'ingrédient à base d'acide aminé est un hydrolysat de protéine.

43. Un procédé selon la revendication 42, dans lequel l'hydrolysat de protéine présente un taux d'hydrolyse qui est d'au moins 5 %.

44. Un procédé selon la revendication 43, dans lequel l'hydrolysat de protéine dérive de l'hémoglobine.

45. Un procédé selon la revendication 34, dans lequel la teneur en ingrédient à base d'acide aminé est comprise entre 5 et 30 % en poids, exprimée par rapport à la composition.

46. Un procédé selon la revendication 34, dans lequel la couche d'enrobage comprend en outre une substance à base d'acide gras en une quantité qui est comprise entre 1 et 50 % en poids exprimée par rapport à la composition.

47. Un procédé selon la revendication 46, dans lequel la quantité de substance à base d'acide gras est comprise entre 5 et 20 % en poids, exprimée par rapport à la composition.

48. Un procédé selon la revendication 34, dans lequel la couche d'enrobage comprend un mélange émulsifié de l'ingrédient à base d'acide aminé et d'une substance à base d'acide gras.

49. Un procédé selon la revendication 34, dans lequel le noyau intérieur enrobé comprend un ingrédient à base de fibre diététique.

50. Un procédé selon la revendication 49, dans lequel au moins 50 % de la fibre diététique de l'ingrédient à base de fibre diététique est soluble dans l'eau.

51. Un procédé selon la revendication 49, dans lequel la teneur en matière formant le noyau interne dans l'Ingrédient à base de fibre diététique est de 1 à 50% en poids, calculée par rapport à la composition.

52. Un procédé selon la revendication 51, dans lequel la teneur en ingrédient à base de fibre diététique est de 5 à 30 % en poids exprimée par rapport à la composition.

53. Un procédé selon la revendication 34, dans lequel la matière de support formant le noyau intérieur comprend un mélange d'un ingrédient à base de fibre diététique et d'un dérivé organique de fer.

54. Un procédé selon la revendication 34, dans lequel le rapport pondéral entre la couche d'enrobage et le noyau interne est de 10/90 à 99/1.

55. Un procédé selon la revendication 54, dans lequel le rapport pondéral précité est compris entre 20/80 et 60/40.
